# NOUVEAU FASCICULE DE BREVET EUROPEEN

(11) **EP 0 412 878 B2**
(45) Date de publication et mention de la décision concernant l'opposition: **04.03.1998**
(45) Mention de la délivrance du brevet: 16.03.1994
(21) Numéro de dépôt: 90402200.1
(22) Date de dépôt: 01.08.1990
(51) Int. Cl.: C03C 13/00, C03C 3/097, C03C 3/089

(54) **Fibres de verre susceptibles de se décomposer en milieu physiologique**
In physiologisches Medium abbaubare Glasfasern
Glass fibres degradable in physiological medium

(30) Priorité: 11.08.1989 FR 8910834; 09.02.1990 FR 9001497
(43) Date de publication de la demande: 13.02.1991
(73) Titulaire: ISOVER SAINT-GOBAIN, F-92400 Courbevoie (FR)
(72) Inventeur: Furtak, Hans, Speyer am Rhein (DE); Tiesler, Hartmut, D-6719 Bockenheim (DE); Cohen,Isabelle, F-73000 Chambéry (FR); Thélohan,Sylvie, F-75014 Paris (FR)
(74) Mandataire: Breton, Jean-Claude

(56) Documents cités:
- EP-A- 9 418
- EP-A- 399 320
- EP-A- 0 019 600
- EP-A- 0 091 866
- EP-A- 0 135 449
- EP-B- 19 600
- EP-B- 231 691
- WO-A-87/05007
- JP-A- 5 077 623
- SU-A- 1 351 896
- US-A- 3 853 569
- CHEMICAL ABSTRACTS, vol. 84, no. 12, 22 mars 1976 Columbus, Ohio, USA page 306; ref. no. 78607G & JP-A-50077623 (A.NISHIKAWA) (25-06-1975)
- CHEMICAL ABSTRACTS, vol. 108, no. 8, février 1988 Columbus, Ohio, USA page 322; ref. no. 61226Y & SU-A-1351896 (ST.SC.RES.INST.GLASS) (15-11-87)
- 1982 Copenhagen Conference "Biological Effects of Man-Made Mineral Fibres", Vol. 2, pages 27-59 de Forster et 87-101 de Leineweber
- Ullmann, 1978, pages 359-365
- Ohta, Ceramic Bulletin, Vol. 57, No. 6, 1978, pages 602-604
- Mitra, Indian Ceramics, Vol. 13, No. 4, Juillet 1968, pages 97-102
- McVay, Materials Research Society Proceedings, Vol. 26, pages 755-761 de Plodinec
- Glasuren und ihre Farben, 1973, Wilhelm Knapp Verlag
- Research Digest, 1954
- Andersson O.A. et al., Glastech, Ber. 61 (1988), Nr. 10

## Description

La présente invention concerne le domaine des fibres de verre ; elle vise plus précisément des fibres de verre dont la composition est telle qu'elles se dégradent dès qu'elles sont en contact d'un milieu physiologique.

L'isolation thermique et acoustique des bâtiments est très souvent réalisée à partir de produits constitués pour l'essentiel de fibres minérales, telles que des fibres de verre. La configuration particulière des lieux à isoler conduit souvent les personnes chargées de la pose de ces produits à les découper sur place. Cette opération provoque la rupture des fibres et, éventuellement, la dispersion de certaines d'entre elles dans l'atmosphère. Il s'ensuit que, parfois, une fibre peut être inhalée accidentellement.

Bien que la nocivité des fibres inhalées n'ait pas été démontrée, le besoin se fait sentir de rassurer les utilisateurs en leur proposant un produit dont l'innocuité est réelle.

Le but de la présente invention est de proposer des fibres de verre dont la composition est telle qu'elles se dégradent rapidement en contact d'un milieu physiologique.

La présente invention a également pour objet de proposer des compositions de verre susceptibles d'être transformées en fibres en utilisant les techniques traditionnelles telles que les techniques de centrifugation.

Les compositions verrières destinées à être transformées en fibres par les techniques de centrifugation dite interne, c'est-à-dire les techniques dans lesquelles le matériau fondu contenu par le centrifugeur s'en échappe par des orifices périphériques de faible dimension, sont parmi celles pour lesquelles les conditions d'utilisation sont les plus contraignantes. Elles doivent notamment pouvoir être travaillées à des températures relativement faibles pour garantir une longévité suffisante du matériel et notamment du centrifugeur. De plus, certaines températures caractéristiques de la dévitrification du verre, comme le liquidus, doivent être nettement inférieures aux températures de fibrage du verre afin de minimiser le risque d'apparition accidentelle de cristaux susceptibles d'obturer les orifices du centrifugeur.

Les buts de l'invention sont atteints en modifiant des compositions connues comprenant pour l'essentiel de la silice, de l'alumine, des oxydes alcalins et alcalino-terreux ainsi que de l'anhydre borique. A partir de telles compositions, les inventeurs ont découvert que la diminution du pourcentage d'alumine, voire la suppression de cet oxyde, jointe à la présence éventuelle du pentoxyde de phosphore permet d'obtenir des verres qui, sous forme de fibres, se dégradent rapidement en milieu physiologique.

Les verres selon l'invention possèdent par ailleurs des propriétés qui, pour les principales d'entre elles, sont proches de celles des verres connus. C'est ainsi qu'ils peuvent être transformés en fibres en utilisant les centrifugeurs classiques. A noter également que les verres selon l'invention, malgré la présence éventuelle de phosphore, peuvent être élaborés dans les fours ordinaires sans provoquer une usure excessive des réfractaires.

Les fibres de verre selon l'invention présentent une composition qui renferme les constituants ci-après, dans les proportions pondérales définies par les limites suivantes :

| | |
|---|---|
| Si0₂ | 57 à 70 % |
| Al₂0₃ | 0 à 5 % |
| Ca0 | 5 à 9 % |
| Mg0 | 0 à 5 % |
| Na₂0 + K₂0 | 13 à 18 % |
| B₂0₃ | 4 à 12 % |
| F | 0 à 1,5 % |
| P₂0₅ | 0 à 4 % |
| Impuretés | < 2 % |

le pourcentage de P₂O₅ étant supérieur à 0,1 % lorsque le pourcentage de Al₂O₃ est égal ou supérieur à environ 1 %.

Les compositions ainsi définies peuvent être élaborées à partir de constituants purs, mais sont généralement obtenues par fusion d'un mélange de matières premières naturelles apportant différentes impuretés.

Le domaine des compositions préférées des fibres selon l'invention est défini par les deux séries de limites pondérales suivantes:

| | | |
|---|---|---|
| SiO₂ | 59 à 68 % | 60 à 68 % |
| Al₂O₃ | 0 à 3 % | 1 à 5 % |
| CaO | 6 à 9 % | 6 à 9 % |
| MgO | 2 à 4 % | 2 à 4 % |
| Na₂O | 14 à 17 % | 14 à 17 % |
| K₂O | 0 à 2 % | 0 à 2 % |
| B₂O₃ | 4 à 11 % | 4 à 11 % |
| F | 0 à 1,5 % | 0 à 1,5 % |
| P₂O₅ | 0 à 3 % | 0,5 à 4 % |

Pour pouvoir être utilisées dans les techniques de centrifugation externe, les compositions selon l'invention présentent avantageusement une viscosité adéquate à une température relativement basse. De préférence, pour ces compositions la viscosité de 1000 poises correspond à une température inférieure à 1200°C et de préférence inférieure à 1150°C.

Une autre caractéristique physique importante pour la production des fibres est la ou les températures liées au phénomène de dévitrification, c'est-à-dire à la formation de cristaux dans la masse vitreuse. Plusieurs températures permettent de caractériser cette dévitrification :
- la température à laquelle la vitesse de croissance des cristaux est maximale,
- la température à laquelle la vitesse de croissance des cristaux devient nulle, appelée couramment température de liquidus.

D'une manière générale, il est souhaitable que l'écart entre la température correspondant à une viscosité de 1000 poises et le liquidus ne soit pas inférieure à environ 50°C.

Les avantages de l'invention sont mis en évidence dans la description détaillée qui suit et qui fait référence à des exemples de réalisation :

### PREMIERE SERIE D'ESSAIS :

Trois compositions utilisées pour la production de fibres sont préalablement essayées pour servir de comparaison dans les tests ultérieurs de dégradabilité des compositions selon l'invention (voir tableau 1). La composition 1 est une composition traditionnelle pour la production de fibres d'isolation notamment parles techniques de centrifugation interne. La composition 2 est une composition usuelle pour les techniques de centrifugation externe. La composition 3 a été utilisée pour des productions par étirage au moyen de courants gazeux.

Pour les essais de dégradabilité en milieu physiologique, les différentes compositions de verre sont étirées mécaniquement à un diamètre de 10 micromètres selon le procédé textile sur une filière de laboratoire à orifice unique.

Les fibres obtenues sont plongées dans une solution qui simule un milieu physiologique tamponné et dont la composition chimique est la suivante (exprimée en g/l) :

| | |
|---|---|
| NaCl | 6,78 |
| NH₄Cl | 0,535 |
| NaHC0₃ | 2,268 |
| NaH₂PO₄H₂0 | 0,166 |
| (Na₃ citrate) 2H₂0 | 0,059 |
| Glycine | 0,450 |
| H₂S0₄ | 0,049 |
| CaCl₂ | 0,022 |

L'essai de dégradabilité par cette solution est conduit dans les conditions suivantes : on plonge 30 milligrammes de fibres dans 30 millilitres de solution maintenue en milieu fermé, à la température de 37°C pendant 3, 10 et 32 jours. A l'issue de chacune de ces périodes on mesure la concentration de la silice dissoute dans la solution ; cette concentration est exprimée en milligrammes par litre.

A titre d'information supplémentaire on mesure également la résistance hydrolytique. Cette mesure est effectuée selon une méthode classique appelée méthode DGG. Cette méthode consiste à plonger 10 grammes de verre broyé, dont la taille des grains est comprise entre 360 et 400 micromètres, dans 100 millilitres d'eau à l'ébullition pendant 5 heures. Après refroidissement rapide, on filtre la solution et on évapore à sec un volume déterminé du filtrat. Le poids de la matière sèche obtenue permet de calculer la quantité de verre dissoute dans l'eau ; cette quantité est exprimée en milligrammes par gramme de verre testé.

Les résultats des mesures de dégradabilité et de DGG sont présentés au tableau n° 2 pour chacune des compositions. On constate que la dégradation des fibres dans la solution d'attaque est très variable d'un verre à l'autre. De ces trois compositions seul le verre n°1 présente une dégradation significative, même si elle reste faible par rapport à celle observée pour les fibres selon l'invention. Les deux autres verres sont très faiblement attaqués.

### SECONDE SERIE D'ESSAIS :

Cette série concerne différentes compositions de fibres de verre selon l'invention. Ces compositions, rassemblées dans le tableau n° 3, correspondent aux verres n° 4 à 11. L'un des verres connus, mentionnés précédemment, est repris à titre comparatif (verre n° 1). A partir de ces verres, des fibres d'un diamètre de 10 micromètres ont été étirées dans les mêmes conditions que celles adoptées lors de la première série d'essais.

La résistance chimique de ces fibres en milieu physiologique ainsi que leur résistance hydrolytique (DGG) ont été mesurées dans des conditions identiques à celles décrites ci-dessus.

Le degré de dégradation des fibres est mesuré en déterminant la concentration de silice dissoute pour différents temps de séjour dans la solution d'attaque qui, pour certaines fibres, ont été de 3, 6 et 10 jours.

Il est important de souligner que la mesure étant effectuée en milieu confiné, il convient de suivre la vitesse de dégradation au cours du temps plus que la valeur atteinte à l'expiration du temps d'essai. En effet, l'attaque par la solution se ralentit car son renouvellement n'est pas assurée. Les concentrations de silice dissoute mesurées à l'issue des temps d'attaque les plus courts traduisent le mieux la faculté pour les fibres de se dégrader en milieu physiologique. Les résultats obtenus sont rassemblés au tableau n° 4.

Les verres n° 4, 5, 7 et 8 illustrent l'influence de P₂0₅ sur la vitesse d'attaque des fibres, dont les compositions renferment le même pourcentage de B₂O₃. Au bout de 3 jours, les verres n° 4 et 5, qui contiennent un pourcentage assez élevé de phosphore, se sont décomposés quatre à cinq fois plus vite que le verre n° 1 servant de référence. A teneur constante en alumine, la vitesse de décomposition des vertes augmente avec la teneur en phosphore ; c'est ce qu'illustre les verres n° 4, 7 et 8.

Les verres n° 5 et 10 présentent le même pourcentage de Al₂O₃ mais renferment des pourcentages différents de P₂O₅. La vitesse de décomposition du verre n° 10 est un peu plus faible que celle du verre n° 5, mais la différence observée n'est pas aussi grande que pourrait le justifier l'écart entre les pourcentages de P₂O₅. Il semble que la plus forte teneur en B₂O₃ du verre n° 10 compense, au moins en partie, la diminution du pourcentage de P₂O₅.

Cette influence de B₂O₃ est confirmée par les verres n° 9 et 11, qui contiennent un fort pourcentage de cet oxyde. Le premier de ces verres, malgré un pourcentage assez élevé de Al₂O₃, présente une bonne vitesse de décomposition. Le second se caractérise par une grande vitesse de décomposition, comparativement au verre n° 1O, qui est due à la fois à la diminution de la teneur de Al₂O₃ et au pourcentage élevé de B₂O₃.

La présence de phosphore dans les verres selon l'invention a toujours pour effet d'augmenter la vitesse de décomposition des fibres en milieu physiologique. Toutefois, on constate que la seule diminution de l'alumine, voire la suppression totale de cet oxyde, peut être la cause d'une vitesse de décomposition élevée. C'est ce que montre le verre n° 6 dénué d'alumine, si ce n'est sous forme d'impureté qui provient des matières premières naturelles apportant d'autres constituants du verre; si la présence de phosphore dans les verres de l'invention est généralement souhaitable, elle n'est pas indispensable lorsque la teneur en alumine n'excède pas environ 1 % en poids. A partir de ce pourcentage, il est préférable que la composition des fibres contienne plus de 0,1 % en poids de pentoxyde de phosphore. A partir de 2 % de Al₂O₃, il est souhaitable que le pourcentage de P₂O₅ soit d'au moins 0,5 % en poids.

Pour éviter une usure accélérée des réfractaires constituant les fours de fusion des verres selon l'invention, il est souhaitable que la teneur en P₂0₅ n'excède pas 4 %. Dans les compositions préférées de l'invention, le pourcentage de cet oxyde demeure égal ou inférieur à environ 3 %, le pourcentage d'alumine n'excédant pas alors environ 3 %.

Les verres selon l'invention possèdent des viscosités et des caractéristiques de dévitrification comparables à celles des verres connus comme le verre n° 1 (voir les tableaux n° 5 et 6).

Ces verres présentent donc l'avantage de pouvoir être transformés en fibres à partir d'installations traditionnelles, comme celles employées dans la technique dite de centrifugation interne. Cette technique est décrite dans de nombreux brevets, tels que les brevets US-3.020.586, US-3.304.164, US-2.949.632 ou US-3.523.774. Cette technique consiste pour l'essentiel à alimenteren verre fondu un centrifugeur muni d'une paroi périphérique percée d'un grand nombre d'orifices. Sous l'action de la force centrifuge le verre fondu passe à travers ces orifices, puis est transformé en fibres sous l'action de jets de gaz chaud.

Les fibres ainsi obtenues permettent d'obtenir des produits fibreux d'excellente qualité aptes à de nombreuses applications. Ainsi, par exemple, les fibres selon l'invention sont avantageusement utilisées sous la forme de feutres ou de panneaux géométriquement bien définis, rigidifiés par un liant polymérisé, ou sous la forme de produits tubulaires destinés à isoler les canalisations. Les fibres selon l'invention peuvent être utilisées également sous forme de matelas cousus sur du carton ou du grillage métallique, sous forme de bourrelet, ou même en vrac par remplissage.

**TABLEAU N° 1**

| **Compositions connues (en pourcentages pondéraux)** | | | |
|---|---|---|---|
| Constituants | Verre n° 1 | Verre n° 2 | Verre n° 3 |
| Si0₂ | 65,01 | 44,50 | 59,00 |
| Fe2₀3 | 0,45 | 3,90 | 0,17 |
| Al₂0₃ | 3,40 | 13,80 | 5,50 |
| Ca0 | 7,00 | 27,80 | 2,00 |
| Mg0 | 2,95 | 7,00 | 0,30 |
| Na₂0 | 15,85 | 1,30 | 11,20 |
| K₂0 | 0,70 | 0,60 | 1,60 |
| B₂0₃ | 4,50 | | 11,00 |
| F | | | 1,00 |
| Ba0 | | | 5,00 |
| Zn0 | | | 3,50 |

**TABLEAU N° 2**

| **Résistance chimique en milieu physiologique et dans l'eau** | | | |
|---|---|---|---|
| Si0₂ en mg/l | Verre n° 1 | Verre n° 2 | Verre n° 3 |
| 3 jours | 19,5 | 1,3 | 3,2 |
| 10 jours | 55,6 | 2,6 | 31,7 |
| 32 jours | 117,6 | 2,8 | 47,1 |
| DGG mg/g | 18,00 | 9,0 | 7,5 |

**TABLEAU N° 3**

| **Compositions en pourcentages pondéraux** | | | | | | | | | |
|---|---|---|---|---|---|---|---|---|---|
| Constituants | Verre n° 1 | Verre n° 4 | Verre n° 5 | Verre n° 6 | Verre n° 7 | Verre n° 8 | Verre n° 9 | verre n°10 | verre n°11 |
| Si0₂ | 65,01 | 61,51 | 65,33 | 69,90 | 64,95 | 63,80 | 59,50 | 64,28 | 60,90 |
| Al₂0₃ | 3,40 | 3,40 | 2,05 | 0,13 | 3,30 | 3,30 | 4,90 | 2,1O | 1,1O |
| Ca0 | 7,00 | 7,00 | 7,00 | 7,00 | 6,90 | 6,90 | 7,OO | 7,OO | 6,90 |
| Mg0 | 2,95 | 2,95 | 3,00 | 2,90 | 2,90 | 2,90 | 2,95 | 2,95 | 2,85 |
| Na₂0 | 15,85 | 15,85 | 15,50 | 15,60 | 15,50 | 15,60 | 13,85 | 15,85 | 15,90 |
| K₂0 | 0,70 | 0,70 | 0,08 | 0,07 | 0,60 | 0,60 | 0,70 | 0,60 | 0,60 |
| B₂0₃ | 4,50 | 4,50 | 4,25 | 4,10 | 4,70 | 4,60 | 9,75 | 5,90 | 1O,20 |
| P₂0₅ | - | 3,40 | 2,45 | - | 1,00 | 2,00 | 1,OO | 1,OO | 1,15 |
| autres | 0,59 | 0,69 | 0,34 | O,30 | 0,15 | 0,30 | 0,35 | 0,32 | 0,40 |

**TABLEAU N° 4**

| **Résistance chimique en milieu physiologique Concentration de Si0**_{**2**} **dissoute (en mg/l)** | | | | | | | | | |
|---|---|---|---|---|---|---|---|---|---|
| Temps attaque | Verre n° 1 | Verre n° 4 | Verre n° 5 | Verre n° 6 | Verre n° 7 | Verre n° 8 | Verre n° 9 | Verre n° 10 | Verre n°11 |
| 3jours | 19,5 | 96,3 | 83,4 | 128,3 | 72,7 | 74,9 | 8O,2 | 75,1 | 105,O |
| 6jours | | | | 149,7 | 106,9 | 104,8 | 103,5 | 105,4 | 128,1 |
| 10jours | 55,6 | 132,7 | 132,7 | 162,6 | 124,1 | 128,3 | 119,4 | 127,6 | 147,6 |
| 32jours | 117,6 | 143,0 | 139,0 | - | - | - | - | - | - |

**TABLEAU N° 5**

| **Températures des viscosités - caractéristiques (en °C)** | | | | | | | | | |
|---|---|---|---|---|---|---|---|---|---|
| Viscosité | Verre n° 1 | Verre n° 4 | Verre n° 5 | Verre n° 6 | Verre n° 7 | Verre n° 8 | Verre n° 9 | Verre n° 10 | Verre n° 11 |
| logn=3 | 1075 | - | 1099 | 1095 | 1092 | 1089 | 1030 | 1061 | 1003 |
| logn=2,5 | 1173 | - | 1201 | 1197 | 1195 | 1191 | 1133 | 1164 | 1090 |

**TABLEAU N° 6**

| **Caractéristiques de dévitrification** | | | | | | | | | |
|---|---|---|---|---|---|---|---|---|---|
| T° (°C) | Verre n° 1 | Verre n° 4 | Verre n° 5 | Verre n° 6 | Verre n° 7 | Verre n° 8 | Verre n° 9 | Verre n° 1O | Verre n° 11 |
| Liquidus | 910 | - | 870 | 930 | 970 | 825 | - | 870 | 850 |
| Vit. max | 830 | - | 780 | 820 | 810 | 800 | - | 800 | 795 |
| Vit. max (µ/mn) | 0,65 | - | 0,05 | 0,51 | 0,19 | 0,11 | - | 0,10 | 0,11 |

**TABLEAU N° 7**

| **Résistance hydrolytique - DGG (en mg/g)** | | | | | | | | |
|---|---|---|---|---|---|---|---|---|
| Verre n° 1 | Verre n° 4 | Verre n° 5 | Verre n° 6 | Verre n° 7 | Verre n° 8 | Verre n° 9 | Verre n° 1O | Verre n° 11 |
| 18,0 | 16,0 | 25,0 | 51,0 | 19,2 | 18,7 | 16,1 | 20,5 | 32 |

## Revendications

1. Fibre de verre susceptible de se décomposer en milieu biologique, ***caractérisée en ce que*** sa composition comprend les constituants ci-après dans les proportions pondérales définies par les limites suivantes :
| | |
|---|---|
| SiO₂ | 57 à 70 % |
| Al₂O₃ | 0 à 5 % |
| CaO | 5 à 9 % |
| MgO | 0 à 5 % |
| Na₂O + K₂O | 13 à 18 % |
| B₂O₃ | 4 à 12 % |
| F | 0 à 1,5 % |
| P₂O₅ | 0 à 4 % |
| Impuretés | < 2 % |
et renferme plus de 0,1 % en poids de pentoxyde de phosphore lorsque le pourcentage pondéral d'alumine est égal ou supérieur à 1 %.

2. Fibre de verre selon la revendication 1, ***caractérisée en ce que*** sa composition renferme au moins 0,5% en poids de pentoxyde de phosphore lorsque le pourcentage pondéral d'alumine est au moins égal à 2%.

3. Fibre de verre selon l'une quelconque des revendications précédentes, ***caractérisée en ce que*** sa composition comprend les constituants ci-après dans les proportions pondérales définies par les limites suivantes :
| | |
|---|---|
| SiO₂ | 59 à 68 % |
| Al₂O₃ | 0 à 3 % |
| CaO | 6 à 9 % |
| MgO | 2 à 4 % |
| Na₂O | 14 à 17 % |
| K₂O | 0 à 2 % |
| B₂O₃ | 4 à 11 % |
| F | 0 à 1,5 % |
| P₂O₅ | 0 à 3 % |

4. Fibre de verre selon l'une des revendications 1 et 2, ***caractérisée en ce que*** sa composition comprend les constituants ci-après dans les proportions pondérales définies par les limites suivantes :
| | |
|---|---|
| SiO₂ | 60 à 68 % |
| Al₂O₃ | 1 à 5 % |
| CaO | 6 à 9 % |
| MgO | 2 à 4 % |
| Na₂O | 14 à 17 % |
| K₂O | 0 à 2 % |
| B₂O₃ | 4 à 11 % |
| F | 0 à 1,5 % |
| P₂O₅ | 0,5 à 4 % |

5. Fibres de verre dont la composition est définie par l'une des revendications précédentes, ***caractérisées en ce qu***'elles sont obtenues selon un procédé de fibrage par centrifugation interne.

6. Produit destiné à l'isolation thermique et/ou acoustique et constitué au moins en partie de fibres de verre, ***caractérisé en ce qu***'au moins une partie desdites fibres présentent une composition chimique telle que définie par l'une quelconque des revendications 1 à 4.

## Claims

1. A glass fibre capable of decomposing in a biological medium, characterised in that its composition comprises the following constituents in the proportions by weight which are defined by the following limits:
| | |
|---|---|
| SiO₂ | 57 to 70 % |
| Al₂O₃ | 0 to 5 % |
| CaO | 5 to 9 % |
| MgO | 0 to 5 % |
| Na₂O + K₂O | 13 to 18 % |
| B₂O₃ | 4 to 12 % |
| F | 0 to 1.5% |
| P₂O₅ | 0 to 4 % |
| Impurities | < 2 % |
and contains more than 0.1% by weight phosphorous pentoxide when the percentage by weight of alumina is equal to or greater than 1%.

2. A glass fibre according to claim 1, characterised in that its composition contains at least 0.5% by weight phosphorous pentoxide when the percentage by weight of alumina is at least equal to 2%.

3. A glass fibre according to either of the preceding claims, characterised in that its composition comprises the following constituents in the proportions by weight as defined by the following limits:
| | |
|---|---|
| SiO₂ | 59 to 68 % |
| Al₂O₃ | 0 to 3 % |
| CaO | 6 to 9 % |
| MgO | 2 to 4 % |
| Na₂O | 14 to 17 % |
| K₂O | 0 to 2 % |
| B₂O₃ | 4 to 11 % |
| F | 0 to 1.5% |
| P₂O₅ | 0 to 3 % |

4. A glass fibre according to one of claims 1 or 2, characterised in that its composition comprises the following constituents in the proportions by weight as defined by the following limits:
| | |
|---|---|
| SiO₂ | 60 to 68 % |
| Al₂O₃ | 1 to 5 % |
| CaO | 6 to 9 % |
| MgO | 2 to 4 % |
| Na₂O | 14 to 17 % |
| K₂O | 0 to 2 % |
| B₂O₃ | 4 to 11 % |
| F | 0 to 1.5% |
| P₂O₅ | 0.5 to 4 % |

5. Glass fibres the composition of which is defined by one of the preceding claims, characterised in that they are obtained by an internal centrifugal fibre-drawing process.

6. A product intended for heat and/or sound insulation and constituted at least in part by glass fibres, characterised in that at least a portion of the said fibres exhibit a chemical composition such as is defined by any one of claims 1 to 4.

## Patentansprüche

1. Glasfaser, die sich im biologischen Medium zersetzen kann, **dadurch gekennzeichnet,** daß ihre Zusammensetzung folgende Bestandteile in den Gewichtsanteilen umfaßt, welche durch die Bereiche
| | |
|---|---|
| SiO₂ | 57 bis 70 % |
| Al₂0₃ | 0 bis 5 % |
| CaO | 5 bis 9 % |
| MgO | O bis 5 % |
| Na₂O + K₂O | 13 bis 18 % |
| B₂O₃ | 4 bis 12 % |
| F | O bis 1,5 % |
| P₂O₅ | 0 bis 4 % |
| Verunreinigungen | < 2 % |
begrenzt sind, und dabei mehr als 0,1 Gew.% Phosphorpentoxid enthält, wenn der Gewichtsanteil des Aluminiumoxids gleich oder größer als 1 % ist.

2. Glasfaser nach Anspruch 1, **dadurch gekennzeichnet,** daß ihre Zusammensetzung wenigstens 0,5 Gew.% Phosphorpentoxid enthält, wenn der Gewichtsanteil des Aluminiumoxids wenigstens gleich 2 % ist.

3. Glasfaser nach Anspruch 1 oder 2, **dadurch gekennzeichnet,** daß ihre Zusammensetzung folgende Bestandteile in den Gewichtsanteilen umfaßt, welche durch die Bereiche
| | |
|---|---|
| SiO₂ | 59 bis 68 % |
| Al₂O₃ | 0 bis 3 % |
| CaO | 6 bis 9 % |
| MgO | 2 bis 4 % |
| Na₂O | 14 bis 17 % |
| K₂O | 0 bis 2 % |
| B₂O₃ | 4 bis 11 % |
| F | 0 bis 1,5 % |
| P₂O₅ | 0 bis 3 % |
begrenzt sind.

4. Glasfaser nach Anspruch 1 oder 2, **dadurch gekennzeichnet,** daß ihre Zusammensetzung folgende Bestandteile in den Gewichtsanteilen umfaßt, welche durch die Bereiche
| | |
|---|---|
| SiO₂ | 60 bis 68 % |
| Al₂O₃ | 1 bis 5 % |
| CaO | 6 bis 9 % |
| MgO | 2 bis 4 % |
| Na₂O | 14 bis 17 % |
| K₂O | 0 bis 2 % |
| B₂O₃ | 4 bis 11 % |
| F | 0 bis 1,5 % |
| P₂O₅ | 0,5 bis 4 % |
begrenzt sind.

5. Glasfasern, deren Zusammensetzung durch einen der vorhergehenden Anspruche definiert ist, **dadurch gekennzeichnet,** daß sie nach einem Verfahren zur Herstellung von Fasern durch Innenschleudern hergestellt sind.

6. Erzeugnis, das zur Wärme- und/oder Schalldammung verwendet wird und wenigstens teilweise aus Glasfasern besteht, **dadurch gekennzeichnet,** daß wenigstens ein Teil dieser Fasern eine solche chemische Zusammensetzung aufweist, wie sie durch einen der Ansprüche 1 bis 4 definiert ist.
